# EUROPEAN PATENT APPLICATION

(11) **EP 1 656 931 A1**
(43) Date of publication of application: **17.05.2006**
(21) Application number: 04292697.2
(22) Date of filing: 15.11.2004
(51) Int. Cl.: A61K 31/00, A61K 31/216

(54) **Compounds which inhibits protein prenylation ( e.g. geranylgeranyltransferase or farnesyltransferase inhibitors) for treating Parkinson's disease**

(71) Applicant: Exonhit Therapeutics SA, 75017 Paris (FR)
(72) Inventor: Schwighoffer, Fabien, 94300 Vincennes (FR); Desire, Laurent, 75014 Paris (FR)
(74) Representative: Becker, Philippe

(57) **Abstract**

The invention relates to compounds and their uses, particularly in the pharmaceutical industry. The invention more specifically relates to new uses of compounds that inhibit the prenylation of proteins, in particular the geranylgeranyl and/or farnesyl modifications of proteins, for treating neurodegeneration involving oxidative stress and, more particularly, Parkinson's disease. The invention also relates to corresponding methods of treatment, and can be used in human subjects for preventive or curative treatment, either alone or in combination with other active agents or treatments.

### Annex to the application documents - subsequently filed sequences listing

SEQUENCE LISTING

<110>  Exonhit Therapeutics SA

<120>  Compounds and Methods for treating Parkinson's Disease

<130>  B0330EP

<140>  EP 04292697.2
<141>  2004-11-15

<160>  16

<170>  PatentIn version 3.1

<210>  1
<211>  515
<212>  DNA
<213>  Target sequence

<400>  1
ggggtagggg tgtagggagt cgtacacaca cacaaccaca cttcgaggcc tggctcttta      60
gaaaaggacg ctcaggaacc ccttggcact cagcagggcg cccaggcctg tgccacacag     120
ctggacacac acagacactg ggaagggggg agctcggtgc ccacgctaat attcagcgtg     180
accagccaga gccaccagca gatgtcaggc agtctggtgg tgtcctcggg ggcacgggtc     240
tccctttctc ccggttagtg ggcgaccggg ccaaggaggg ggagccacgc tggcaagggc     300
gtgaggcagg gcgcggcctt catagcacct tgcagcagtt gatgcagcca ttctgggatc     360
cgtagcgctt ctgcagcgcg gcgcgcgtgg ccgtctcgaa aacctcgcgc acgccctcct     420
tggtcttggc cgagcactcg aggtagtcat aggcttggat gcgcaccgcc atggcgcggc     480
cgtcatccgt gcgcactggc tcctgcttca tgcgg                                515

<210>  2
<211>  518
<212>  DNA

<213>  Target sequence

<400>  2
tgtggggtag gggtgtaggg agtcgtacac acacacaacc acacttcgag gcctggctct      60
ttagaaaagg acgctcagga accccttggc actcagcagg gcgcccaggc ctgtgccaca     120
cagctggaca cacacagaca ctgggaaggg gggagctcgg tgcccacgct aatattcagc     180
gtgaccagcc agagccacca gcagatgtca ggcagtctgg tggtgtcctc gggggcacgg     240
gtctcccttt ctcccggtta gtgggcgacc gggccaagga gggggagcca cgctggcaag     300
ggcgtgaggc agggcgcggc cttcatagca ccttgcagca gttgatgcag ccattctggg     360
atccgtagcg cttctgcagc gcggcgcgcg tggccgtctc gaaaacctcg cgcacgccct     420
ccttggtctt ggccgagcac tcgaggtagt cataggcttg gatgcgcacc gccatggcgc     480
ggccgtcatc cgtgcgcact ggctcctgct tcatgcgg                              518

<210>  3
<211>  515
<212>  DNA
<213>  Target sequence

<400>  3
ggcagcgtgt cattaaggag gctgtgctgt gtcacacggt ctgggagcta cgggagggtc      60
tgcacccctg agcccagaag ctgcagtctt cttaaggaca aagtctctca acagcttagt     120
gcttacgtgt tctcagcaca acgcaactta gttcacaagg tattttggca attcttaatc     180
tgagcaagaa taggggattt tgaaaaataa ggctttaaga aggctttgtc attttagggc     240
tgaattttaa tagactgtaa gttggaattc ttacatttaa aacagaacct taaaattact     300
gactggttca ttggttcaaa atggttttat ggaaaaatta atctgtaaca aaaacttggc     360
atcagacgcg aagactccgc cattttctga gcaaagcgta caaaggctcc agggaccaag     420
accgagggag gggctcagac atttacaaca gcaggcattt tctcttcctc ttcttaacag     480
gagggggaca gagaacggct cggatagctt catca                                 515

<210>  4
<211>  507
<212>  DNA
<213>  Target sequence

<400>  4
caggggctag ccatggcgaa agagatcggt gctgtcaaat acctggagtg ctcagcactc      60

acacagcgag gactcaagac agtgtttgat gaagctatcc gagccgttct ctgtccccct     120

cctgttaaga cgaggaagag aaaatgcctg ctgttgtaaa tgtctgagcc cctccctcgg     180

tcttggtccc tggagccttt gtacgctttg ctcagaaaat ggcggagtct tcgcgtctga     240

tgccaagttt ttgttacaga ttaatttttc cataaaacca ttttgaacca atgaaccagt     300

cagtaatttt aaggttctgt tttaaatgta agaattccaa cttacagtct attaaaattc     360

agccctaaaa tgacaaagcc ttcttaaagc cctatttttc aaaatcccct attcttgctc     420

agattaagaa ttgccaaaat accttgtgaa ctaagttgcg ttgtgctgag aacacgtaag     480

cactaagctg ttgagagact ttgtcct                                         507

<210>  5

<211>  524

<212>  DNA

<213>  Target sequence

<400>  5
tacccgcagg ggctagccat ggcgaaagag atcggtgctg tcaaatacct ggagtgctca      60

gcactcacac agcgaggact caagacagtg tttgatgaag ctatccgagc cgttctctgt     120

ccccctcctg ttaagacgag gaagagaaaa tgcctgctgt tgtaaatgtc tgagcccctc     180

cctcggtctt ggtccctgga gcctttgtac gctttgctca gaaaatggcg gagtcttcgc     240

gtctgatgcc aagtttttgt tacagattaa tttttccata aaaccatttt gaaccaatga     300

accagtcagt aattttaagg ttctgtttta aatgtaagaa ttccaactta cagtctatta     360

aaattcagcc ctaaaatgac aaagccttct taaagcccta tttttcaaaa tcccctattc     420

ttgctcagat taagaattgc caaaatacct tgtgaactaa gttgcgttgt gctgagaaca     480

cgtaagcact aagctgttga gagactttgt ccttaagaag actg                      524

<210>  6

<211>  515

<212>  DNA

<213>  Target sequence

<400>  6
tggcagcgtg tcattaagga ggctgtgctg tgtcacacgg tctgggagct acgggagggt      60

ctgcacccct gagcccagaa gctgcagtct tcttaaggac aaagtctctc aacagcttag     120

tgcttacgtg ttctcagcac aacgcaactt agttcacaag gtattttggc aattcttaat 180

ctgagcaaga ataggggatt ttgaaaaata aggctttaag aaggctttgt cattttaggg 240

ctgaatttta atagactgta agttggaatt cttacattta aaacagaacc ttaaaattac 300

tgactggttc attggttcaa aatggtttta tggaaaaatt aatctgtaac aaaaacttgg 360

catcagacgc gaagactccg ccattttctg agcaaagcgt acaaaggctc cagggaccaa 420

gaccgaggga ggggctcaga catttacaac agcaggcatt ttctcttcct cttcttaaca 480

ggagggggac agagaacggc tcggatagct tcatc 515

<210> 7

<211> 523

<212> DNA

<213> Target sequence

<400> 7
tggcagcgtg tcattaagga ggctgtgctg tgtcacacgg tctgggagct acgggagggt 60

ctgcacccct gagcccagaa gctgcagtct tcttaaggac aaagtctctc aacagcttag 120

tgcttacgtg ttctcagcac aacgcaactt agttcacaag gtattttggc aattcttaat 180

ctgagcaaga ataggggatt ttgaaaaata aggctttaag aaggctttgt cattttaggg 240

ctgaatttta atagactgta agttggaatt cttacattta aaacagaacc ttaaaattac 300

tgactggttc attggttcaa aatggtttta tggaaaaatt aatctgtaac aaaaacttgg 360

catcagacgc gaagactccg ccattttctg agcaaagcgt acaaaggctc cagggaccaa 420

gaccgaggga ggggctcaga catttacaac agcaggcatt ttctcttcct cttcttaaca 480

ggagggggac agagaacggc tcggatagct tcatcaaaca ctg 523

<210> 8

<211> 192

<212> PRT

<213> Target sequence

<400> 8

Met Gln Ala Ile Lys Cys Val Val Val Gly Asp Gly Ala Val Gly Lys
1               5                   10                  15

Thr Cys Leu Leu Ile Ser Tyr Thr Thr Asn Ala Phe Pro Gly Glu Tyr
            20                  25                  30

Ile Pro Thr Val Phe Asp Asn Tyr Ser Ala Asn Val Met Val Asp Gly
        35                  40                  45

Lys Pro Val Asn Leu Gly Leu Trp Asp Thr Ala Gly Gln Glu Asp Tyr
    50                  55                  60

Asp Arg Leu Arg Pro Leu Ser Tyr Pro Gln Thr Asp Val Phe Leu Ile
65                  70                  75                  80

Cys Phe Ser Leu Val Ser Pro Ala Ser Phe Glu Asn Val Arg Ala Lys
                85                  90                  95

Trp Tyr Pro Glu Val Arg His His Cys Pro Asn Thr Pro Ile Ile Leu
            100                 105                 110

Val Gly Thr Lys Leu Asp Leu Arg Asp Asp Lys Asp Thr Ile Glu Lys
        115                 120                 125

Leu Lys Glu Lys Lys Leu Thr Pro Ile Thr Tyr Pro Gln Gly Leu Ala
    130                 135                 140

Met Ala Lys Glu Ile Gly Ala Val Lys Tyr Leu Glu Cys Ser Ala Leu
145                 150                 155                 160

Thr Gln Arg Gly Leu Lys Thr Val Phe Asp Glu Ala Ile Arg Ala Val
                165                 170                 175

Leu Cys Pro Pro Pro Val Lys Lys Arg Lys Arg Lys Cys Leu Leu Leu
            180                 185                 190

<210> 9

<211> 196

<212> PRT

<213> Target sequence

<400> 9

Met Ala Ala Ile Arg Lys Lys Leu Val Val Val Gly Asp Gly Ala Cys
1               5                   10                  15

Gly Lys Thr Cys Leu Leu Ile Val Phe Ser Lys Asp Glu Phe Pro Glu
            20                  25                  30

Val Tyr Val Pro Thr Val Phe Glu Asn Tyr Val Ala Asp Ile Glu Val
        35                  40                  45

Asp Gly Lys Gln Val Glu Leu Ala Leu Trp Asp Thr Ala Gly Gln Glu

50                  55                  60

Asp Tyr Asp Arg Leu Arg Pro Leu Ser Tyr Pro Asp Thr Asp Val Ile
65                  70                  75                  80

Leu Met Cys Phe Ser Val Asp Ser Pro Asp Ser Leu Glu Asn Ile Pro
                85                  90                  95

Glu Lys Trp Val Pro Glu Val Lys His Phe Cys Pro Asn Val Pro Ile
            100                 105                 110

Ile Leu Val Ala Asn Lys Lys Asp Leu Arg Ser Asp Glu His Val Arg
        115                 120                 125

Thr Glu Leu Ala Arg Met Lys Gln Glu Pro Val Arg Thr Asp Asp Gly
    130                 135                 140

Arg Ala Met Ala Val Arg Ile Gln Ala Tyr Asp Tyr Leu Glu Cys Ser
145                 150                 155                 160

Ala Lys Thr Lys Glu Gly Val Arg Glu Val Phe Glu Thr Ala Thr Arg
                165                 170                 175

Ala Ala Leu Gln Lys Arg Tyr Gly Ser Gln Asn Gly Cys Ile Asn Cys
            180                 185                 190

Cys Lys Val Leu
        195

<210>  10

<211>  515

<212>  DNA

<213>  Target sequence

<400>  10
cattaatggt gagcgtcgta aatactgaaa catcattagg tttgagcacc accttttctc      60

ctccagagtt catcttgaca atgatcaggc cttccccaga gccaagtttg tctgctactg     120

cactgatgac ttccttcacc gaggcggcta caggcactcg gatggtggta taggtgtggt     180

caatgcagta gaccttgaac aaaacctcat cagagccacg aatcggctga cgcttctggg     240

ccctctcgtc acctgtatta aactgctgca aaagcacctt gtgcttcttc tgtggagctt     300

ttgcgtcttc tgagatctgt ttgacagtct tctccagctc agccagctgc tccttgaagg     360

cgaccatcat ccgtgcatca tctgatacag acacatagaa ctcctccagg aaagccatgg     420

ccacgtcgtc ttcttggaga agatcaccat acatggctgc ccattgcaga accaggcggg     480

tgacccgcct cttgttgttg agggcgtaat ccatt 515

<210> 11

<211> 482

<212> DNA

<213> Target sequence

<400> 11
cgtaaatact gaaacatcat taggtttgag caccaccttt tctcctccag agttcatctt 60

gacaatgatc aggccttccc cagagccaag tttgtctgct actgcactga tgacttcctt 120

caccgaggcg gctacaggca ctcggatggt ggtataggtg tggtcaatgc agtagacctt 180

gaacaaaacc tcatcagagc cacgaatcgg ctgacgcttc cgggccctct cgtcacctgt 240

attaaactgc tgcaaaagca ccttgtgctt cttctgtgga gcttttgcgt cttctgagat 300

ccgtttgaca gtcttctcca gctcagccag ctgctccttg aaggcgacca tcatccgtgc 360

atcatctgat acagacacat agaactcctc caggaaagcc atggccacgt cgtcttcttg 420

gagaagatca ccatacatgg ctgcccattg cagaaccagg cgggtgaccc gcctcttgtt 480

gt 482

<210> 12

<211> 521

<212> DNA

<213> Target sequence

<400> 12
aaacaggcgt ccattaatgg tgagcgtcgt aaatactgaa acatcattag gtttgagcac 60

caccttttct cctccagagt tcatcttgac aatgatcagg ccttccccag agccaagttt 120

gtctgctact gcactgatga cttccttcac cgaggcggct acaggcactc ggatggtggt 180

ataggtgtgg tcaatgcagt agaccttgaa caaaacctca tcagagccac gaatcggctg 240

acgcttctgg gccctctcgt cacctgtatt aaactgctgc aaaagcacct tgtgcttctt 300

ctgtggagct tttgcgtctt ctgagatctg tttgacagtc ttctccagct cagccagctg 360

ctccttgaag gcgaccatca tccgtgcatc atctgataca gacacataga actcctccag 420

gaaagccatg gccacgtcgt cttcttggag aagatcacca tacatggctg cccattgcag 480

aaccaggcgg gtgacccgcc tcttgttgtt gagggcgtaa t 521

<210> 13

<211>  476

<212>  DNA

<213>  Target sequence

<400>  13
tgtcttggta ctggagaagg tcccagcaag gaacagagct tctaatcaag gaaactcaca      60

gcctcagcaa aagtatactg tgatgtcagg aacacctgaa aagattttag agcattttct     120

agaaacaata cgccttgagc catctttgaa tgaagcaaca gattcagttt taaatgactt     180

cgttatgatg cactgtgttt ttatgccaaa cacccagctt tgccccgccc tcgtggccca     240

ttaccacgca cagccttctc aaggtacaga acaggagaga atggattacg ccctcaacaa     300

caagaggcgg gtcacccgcc tggttctgca atgggcagcc atgtatggtg atcttctcca     360

agaagacgac gtggccatgg ctttcctgga ggagttctat gtgtctgtat cagatgatgc     420

acggatgatg gtcgccttca aggagcagct ggctgagctg gagaagactg tcaaac          476

<210>  14

<211>  507

<212>  DNA

<213>  Target sequence

<400>  14
gaatacagtc agacttaaag aacatgacca agatgtcttg gtactggaga aggtcccagc      60

aaggaacaga gcttctaatc aaggaaactc acagcctcag caaaagtata ctgtgatgtc     120

aggaacacct gaaaagattt tagagcattt tctagaaaca atacgccttg agccatcttt     180

gaatgaagca acagattcag ttttaaatga cttcgttatg atgcactgtg tttttatgcc     240

aaacacccag ctttgccccg ccctcgtggc ccattaccac gcacagcctt ctcaaggtac     300

agaacaggag agaatggatt acgccctcaa caacaagagg cgggtcaccc gcctggttct     360

gcaatgggca gccatgtatg gtgatcttct ccaagaagac gacgtggcca tggctttcct     420

ggaggagttc tatgtgtctg tatcagatga tgcacggatg atggtcgcct tcaaggagca     480

gctggctgag ctggagaaga ctgtcaa                                         507

<210>  15

<211>  506

<212>  DNA

<213>  Target sequence

<400>  15
gaatacagtc agacttaaag aacatgacca agatgtcttg gtactggaga aggtcccagc      60

aaggaacaga gcttctaatc aaggaaactc acagcctcag caaaagtata ctgtgatgtc     120

aggaacacct gaaaagattt tagagcattt tctagaaaca atacgccttg agccatcttt     180

gaatgaagca acagattcag ttttaaatga cttcgttatg atgcactgtg tttttatgcc     240

aaacacccag ctttgccccg ccctcgtggc ccattaccac gcacagcctt ctcaaggtac     300

agaacaggag agaatggatt acgccctcaa caacaagagg cgggtcaccc gcctggttct     360

gcaatgggca gccatgtatg gtgatcttct ccaagaagac gacgtggcca tggctttcct     420

ggaggagttc tatgtgtctg tatcagatga tgcacggatg atggtcgcct tcaaggagca     480

gctggctgag ctggagaaga ctgtca                                          506

<210>  16

<211>  522

<212>  DNA

<213>  Target sequence


<400>  16
tggagagagg cttggcaatc ttcaggaatt gatgctgcag tagctcttta gctgaacctc      60

tcttctccac atccatctca agacagcggt tcaaaaagtc ccggaaaata gctgacaact     120

tctctgggtt ctgaagctct ggcgtcccat tggtagcaat gaggtacaag gctctcaaag     180

ggttttcatt gaggtatggg ggctccccct caatcatttc aattgccata atacccaggg     240

accagatgtc aaccttgggt ccataggcct tgcgtgtcac aacttcaggt gccatccaat     300

atggagttcc caccatggtg ctccttttgc tctgctctgg agttatctgt gcacagaatc     360

caaagtcagt taatttgaca gagccatcca ttcccagcag aatattgtca ctcttgatgt     420

ctctgtgaat gacttggttt gaatgcagga actccaaagc ttgtagacac tctctgcaca     480

cagctgctat ctggcctcta ctgataacgc ttctcaaggg cg                        522

## Description

The invention relates to compounds and their uses, particularly in the pharmaceutical industry. The invention more specifically relates to new uses of compounds that inhibit the prenylation of proteins, in particular the geranylgeranyl and/or farnesyl modifications of proteins, for treating neurodegeneration involving oxidative stress and, more particularly, Parkinson's disease. The invention also relates to corresponding methods of treatment, and can be used in human subjects for preventive or curative treatment, either alone or in combination with other active agents or treatments.

### BACKGROUND

Parkinson's disease (PD) is a progressive neurodegenerative disorder primarily characterized by muscular rigidity, tremor and abnormalities of posture. This emphasis on the motor disorder has overshadowed the cognitive and behavioral consequences of this disease. For instance, PD symptoms include a high incidence of depression and anxiety, and as many as 30% of all PD patients will experience dementia (Louis et al. 2004; Anderson 2004).

The pathological hallmark of PD is the degeneration of dopaminergic neurons. However, the neuronal loss is more widespread and affects other area of the brain, like the prefrontal cortex, which accounts for the non motor symptoms (Olanow and Tatton 1999). Oxidative stress is the central phenomenon leading to neuronal death in PD (Tabner et al. 2001).

CAAX prenyltransferases, e.g., protein farnesyltransferase (FTase) and geranylgeranyltransferase (GGTase) catalyze the posttranslational attachment of an isoprenoid lipid group (prenylation) to many signal transduction proteins, including members of the Ras GTPase superfamily. Since the discovery that the farnesylation of Ras oncoproteins (which are associated with up to a quarter of all human cancers including 90% of all pancreatic cancers and 50% of colon cancers) is essential for their transforming activity, FTase has emerged as a major anti-cancer drug target. Inhibitors of FTase (FTIs) can cause tumor regression in animals and are being evaluated in clinical trials (Phase I, II, III) for the treatment of human cancer. A large number of articles and applications have been published, relating to prenyltransferase inhibitors for use against cancers, and at least six of these inhibitors are in clinical trials.

### SUMMARY OF THE INVENTION

The present invention now surprisingly and unexpectedly demonstrates that such prenyl inhibitors exhibit potent activity against oxidative stress, and particularly in the treatment of Parkinson's Disease.

The present invention represents the first report of the potent activity of prenylation inhibitors (e.g., GGT or FT inhibitors) against Parkinson's Disease and allows the development of novel and effective therapeutic approaches of this progressive and severe neurodegenerative disease.

In this regard, a particular object of this invention resides in the use of a protein prenylation inhibitor, particularly a GGT or FT inhibitor, for the manufacture of a medicament for treating Parkinson's Disease.

A further object of this invention resides in the use of a protein prenylation inhibitor, particularly a GGT or FT inhibitor, for the manufacture of a medicament for protecting neurons from oxidative stress in subjects having Parkinson's Disease.

An other object of this invention resides in the use of a protein prenylation inhibitor, particularly a GGT or FT inhibitor, for the manufacture of a medicament for protecting dopaminergic neurons in a subject having Parkinson's Disease.

An other aspect of this invention is a method of treating Parkinson's Disease, comprising administering to a subject in need thereof an effective amount of a protein prenylation inhibitor, particularly a GGT or FT inhibitor.

According to particular embodiments of the invention, the inhibitor is a compound having an IC50 for GGT or FT that is below about 1 mM, and/or selective for FT or GGT and/or that crosses the blood-brain barrier and/or having a molecular weight below about 800 daltons. Typical examples of such inhibitors include are provided in the present application, including L-744,832.

The invention can be used in human subjects for preventive or curative treatment, either alone or in combination with other active agents or treatments.

### LEGEND TO THE FIGURES

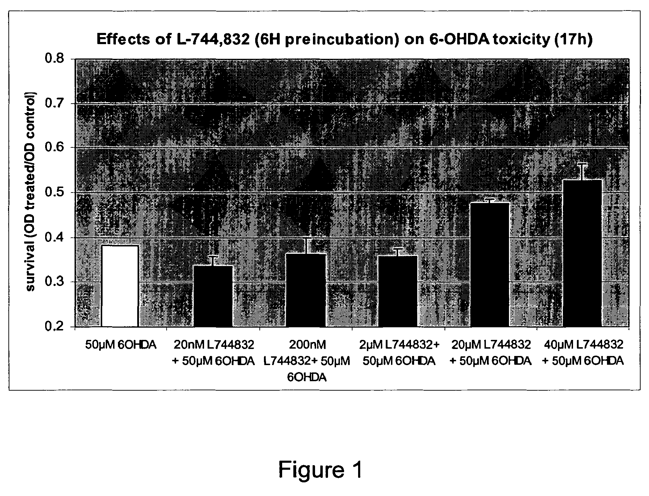

Figure 1: Following an overnight treatment with 6-OHDA, L-744,832 exhibits a significant protective effect against cell death induced by ROS.

### DETAILED DESCRIPTION OF THE INVENTION

As indicated above, the present invention relates to the use of protein prenylation inhibitors, particularly GGT or FT inhibitors, for treating Parkinson's Disease.

In order to identify pathways and targets to enable the discovery of new compounds for Parkinson's Disease ("PD") treatments, the inventors applied DATAS to dopaminergic neurons exposed to oxidative stress induced by the 1-methyl-4-phenyl-1,2,3,6-trtrahydropyridine (MPTP) toxin. This toxin can induce PD pathology and symptoms in animal models and in human. DATAS is a patented gene profiling technology (U.S. Pat. No. 6,251,590), which allows the systematic analysis of transcripts that are differentially spliced between two physiopathological situations. Based on the identification of splicing alterations induced by MPTP exposure, several unprecedented pathways, receptors and enzymes were identified.

Among the pathways identified was a signaling cascade involving the following molecular players:
- Rap guanine nucleotide exchange factor (GEF) 4
- RhoB gene (Arhb)
- Rac1
- p21/Cdc42/Rac1-activated kinase 1 (STE20 homolog, yeast) (PAK1) This pathway is involved in the regulation of cell viability and cytoskeleton organization (Ridley 2001; Aznar and Lacal 2001).

Rac1 is involved in NADPH activation and therefore plays a role in oxidative stress. Our identification of differentially regulated splicing of the Rac1 pathway during the intoxification of dopaminergic neurons with MPTP provides the first evidence of the involvement of Rac1 in the oxidative stress of dopaminergic neurons. Inhibiting the Rac1 protein and pathway represents a new therapeutic approach to rescue and protect dopaminergic neurons from oxidative stress and more precisely from the oxidative stress induced neurotoxicity observed in a disease like Parkinson's Disease.

Rac1 is a member of the small GTPase (SMG) protein family, which are monomeric guanine nucleotide-binding proteins of 20-25 kDa molecular mass that function as molecular switches. They are "on" in the GTP-bound state and "off" in the GDP-bound state. Cycling between the active and inactive forms is controlled by several accessory proteins: the guanine nucleotide exchange factors (GEFs), GTPase-activating proteins (GAPs) and GDP dissociation inhibitors (GDIs). The active GTP-bound GTPases interact with a variety of effector proteins to produce their cellular effects.

The GTP-bound form of Rac1 needs to be post-translationally modified via several enzymatic steps. One of them, which is mandatory for Rac1 activity, is the geranylgeranylation of its C-terminal end. This modification is triggered by geranylgeranyltransferases enzymes (GGT).

RhoB, which is also regulated by alternative splicing modification during the toxic induction of dopaminergic neurons with MPTP, is part of and involved in the regulation of the Rac1 pathway. RhoB has been described as an early predictor of neuronal death, in vivo, during brain ischemia (Trapp et al. 2001).

The present invention, for the first time, provides evidence that RhoB modifications are linked to the neuronal death induced by oxidative stress and delineates a rationale for inhibiting RhoB in order to protect neurons in PD . RhoB is another member of the small GTPase family. Its activity also requires post translational modifications involving several enzymatic steps. One of them, which is mandatory for RhoB activity, is the farnesylation of its C-terminal end (Crul et al. 2001). This modification is facilitated by farnesyltransferases enzymes (FT).

For the first time, the invention thus provides evidence that GGT inhibitors and FT inhibitors represent potent compounds for the protection of neurons against oxidative stress, more precisely for treating Parkinson's disease. The experimental section further documents the neuroprotective activity of such compounds, thus confirming the proposed therapeutic utility.

### GGT or FT inhibitors

Within the context of this invention, a protein prenylation inhibitor designates any compound, agent or treatment that inhibits (e.g., reduces or abolishes) the prenylation of proteins, more specifically the prenylation of SMG proteins. Such inhibitors include more specifically any compound (e.g., antagonist) that inhibits a prenylation enzyme, particularly a prenyl-transferase enzyme, more particularly a CAAX-prenyltransferase. Specific and preferred examples of such enzymes include GeranylGeranylTtransferase(s) ("GGT") and farnesyltransferase(s) ("FT").

In a preferred embodiment, the FT inhibitors ("FTIs") or GGT inhibitors ("GGTIs") have an IC50 for the FT or GGT, respectively, which is below 1 mM and, more preferably, below 50 nM.

Furthermore, preferred FTIs or GGTIs can get through (i.e., cross) the blood-brain barrier (BBB). In this regard, the FTls or GGIs to be used in the present invention generally present a molecular weight less than about 800 daltons, preferably less than about 600 daltons.

The inhibitors can inhibit either GGT or FT, or both (i.e., dual inhibitors). Alternatively, a combination comprising a GGT inhibitor and a FT inhibitor can be used.

Most preferred GGT or FT inhibitors are selective inhibitors, i.e., they are essentially active on GGT or FT with no substantial specific activity on other enzymes.

Most preferred FT inhibitors for use in the present invention are listed below:
6-[Amino(4-chlorophenyl)-1-methyl-1H-imidazol-5-ylmethyl]-4-(3-chlorophenyl)-1-methyl-2(1H)-quinolinone (also identified as R115777, Tipifarnib or ZarnestraTM, whose FTase IC50 is 0.86 nM)
4-(3-chlorophenyl)-6-[(4-chlorophenyl)hydroxy(1-methyl-1H-imidazol-5-yl)methyl]-1-methyl-2(1H)-quinolinone,
6-[(4-chlorophenyl)hydroxy(1-methyl-1H-imidazol-5-yl)methyl]-4-(3-ethoxyphenyl)-1-methyl-2(1H)-quinolinone,
6-[(4-chlorophenyl)(1-methyl-1H-imidazol-5-yl)methyl]-4-(3-ethoxyphenyl)-1-methyl-2(1 H)-quinolinone monohydrochloride monohydrate,
6-[amino(4-chlorophenyl)(1-methyl-1 H-imidazol-5-yl)methyl]-4-(3-ethoxyphenyl)-1-methyl-2(1H)-quinolinone,
6-[amino(4-chlorophenyl)(1-methyl-1H-imidazol-5-yl)methyl]-1-methyl-4-(3-propylphenyl)-2(1H)-quinolinone,
(B)-6-[amino(4-chlorophenyl)(1-methyl-1H-imidazol-5-yl)methyl]-4-(3-chlorophenyl)-1-methyl-2(1H)-quinolinone, the above cited compounds are described in patent applications WO9716443 and EP1162201 (Janssen Pharmaceutica NV (BE) - Johnson & Johnson). R115777 presents the following formula: 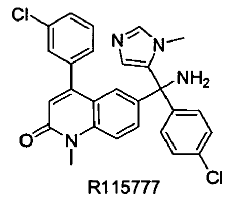
(+)-4-[2-[4-(8-Chloro-3,10-d ibromo-6,11-d ihydro-5H-benzo-[5,6]cyclohepta[1,2-b]-pyridin-11(R)-yl)-1 -piperidiny!]-2-oxo-ethyt]-1 -piperidinecarboxamide (also identified as Sch-66336, Lonafarnib, SCH 66336 or SarasarTM, whose FTase IC50 is 1.9 nM) ; described in US5874442 (Schering Corp. (US) - Schering-Plough). SCH-66336 presents the following formula: 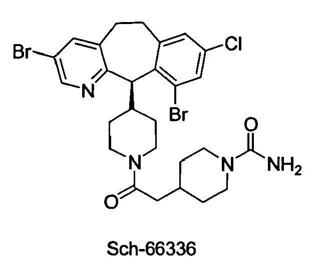
(R)-7-Cyano-2,3,4,5-tetrahydro-1-(1H-imidazol-4-ylmethyl)-3-(phenylmethyl)-4-(2-thienylsulfonyl)-1H-1,4-benzodiazepine (also identified as BMS-214662, whose FTase IC50 = 0.7 nM). This compound is described in Hunt, J. T. et al., J. Med. Chem. 2000, 43, 3587-3595. BMS-214662 presents the following formula: 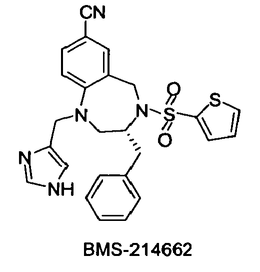
Isopropyl (2S)-2-({2-(4-fluorophenetyl)-5-[({(2S,4S)-4-[(3-pyridinylcarbonyl)sulfanyl]tetrahydro-1 H-pyrrol-2-yl}methyl)amino]benzoyl}amino)-4-(methylsufanyl)butanoate, also identified as AZD-3409 and described in WO0146137.(AstraZeneca).
2,3,4,5-Tetrahydro-1-(1H-imidazol-4-ylmethyl)-4-(1-naphthalenylcarbonyl)-1H-1,4-benzodiazepine, hydrochloride; this compound is described in WO9730992 (Squibb Bristol Myers Co (US)). The above compound has the following formula: 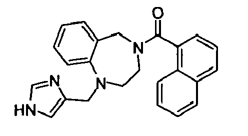
1-(3-Chlorophenyl)-4-[1-(4-cyanobenzyl)-5-imidazolylmethyl]-2-piperazinone (also identified as L-778,123, FTase IC50 = 2 nM and described in Lobell, R. B., Mol. Cancer Ther., 2002, 1, 747 (Merck & Co). L-778,123 has the following formula: 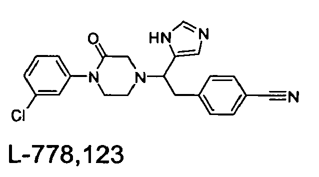
1(R),10(S)-Epoxy-5(S),5(S),7(S)-guaia-3(4),11(13)-dien-6,12-olide ,also identified as Arglabin, and described in WO9848789 (Paracure Inc. (US) - Nuoconlogy Labs).
L-Methionine, N-[[(4R)-3-[(2S,3S)-2-[[(2R)-2-amino-3-mercaptopropyl]amino]-3-methylpentyl]-5,5-dimethyl-4-thiazolidinyl]carbonyl]-, methyl ester, also identified as BIM-46068, and described in WO9800409 (Biomeasure Inc. (US) - lpsen.
L-Methionine, N-[[5-[[(1 H-imidazol-4-ylmethyl)amino]methyl]-2'-methyl[1,1'-biphenyl]-2-yl]carbonyl] or also called FTI-2148 and its methyl ester (FTI-2153), described in WO9717070 (Pittsburgh University - Abbott).
4-[(4-Cyano-2-arylbenzyloxy)-(3-methyl-3H-imidazol-4-yl)methyl]benzonitriles, referred as A315493 and A313326, and 5-cyano-2-[(4-cyanophenyl)-(3-methyl-3H-imidazol-4-yl)methoxymethyl]-N-phenylbenzamides described in Wang L. et al., J. Med. Chem., 47, 612, 2004 (Abbott) (A315493 FTase IC50 = 0.4 nM and GGTase I = 24 nM, A315326 FTase IC50 = 0.3 nM and GGTase I = 18 nM). These compounds present the following formulas: 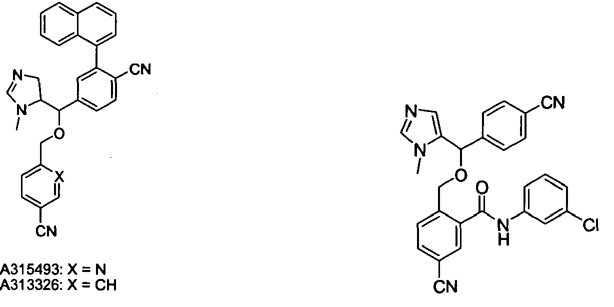
FTI-276 and FTI-277 described in Lerner E. C. et al., J. Biol. Chem., 270, 45, 26770, 1995 and Lerner E. C. et al., J. Biol. Chem., 270, 45, 26802, 1995. (Pittsburgh University), with FTI-276 PTFase IC50 = 0.5 nM and FTI-277 PTFase IC50 = 100 nM. These compounds have the following formulas: 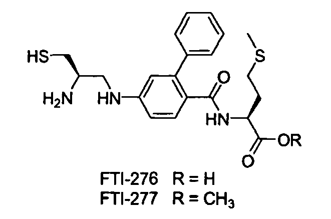
(2S)-2-[[(2S)-2-[[(2S,3S)-2-[[(2R)-2-Amino-3-mercaptopropyl]amino]-3-methylpentyl]oxy]-1-oxo-3-phenylpropyl]amino]-4-(methylsulfonyl)-butanoic acid 1-methylethyl ester, also identified as L-744,832 and described in Law, B.K., et al., J. Biol. Chem. 275, 10796, 2000 (Merck & Co). L-744,832 presents the following formula: 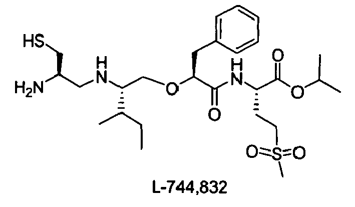
1-[1-[1-(1,3-Benzodioxol-5-ylmethyl)-1H-imidazol-5-ylmethyl]-4-(1-naphthyl)-1H-pyrrol-3-yl]-1-(4-methyl-1-piperazinyl)methanone, also called LB-42908, described in WO9928315 (LG Chemical Ltd (US) - LG Life Sciences).
2-(3-Pyridyl)-N-(2,2-diphenyl-ethyl)-N-((cis)-3-sulfanylpyrrolidin-2-ylmethyl)acetamide described in WO9807692 (Zeneca Ltd (GB) - AstraZeneca).
(7,8-Dichloro-5H-dibenzo[b,e][1,4]diazepin-11-yl)-pyridin-3-yl methylamine described in WO9700252 (Wamer Lambert Co (US) - Pfizer).
(2 alpha)-2-Hydroxy-24,25-dihydroxylanost-8-en-3-one or clavarinone and clavaric acid and lanost-8,24-dien-3-one described in WO9635707 (Merck & Co Inc. (US)).
L-erythro-L-Glycero-D-altro-7-trideculo-7,4-furanosonic acid, 2,7-anhydro-3,4-di-C-carboxy-8,9,10,12,13-pentadeoxy-10-methylene-12-(phenylmethyl)-,11-acetate 5-(4,6-dimethyl-2-octenoate), [5(2E,4S,6S),7S] or zaragozic acid A described in WO9404144 (Merck & Co Inc. (US)) (Zaragozic acid PFTase IC50 = 50 nM).
2,4-Decadienamide, N-(5-hydroxy-5-(7-((2-hydroxy-5-oxo-1-cyclopenten-I-yl)amino-oxo-1,3,5-heptatrienyl)-2-oxo-7-oxabicyclo(4.1.0)hept-3-en-3-yl)-2,4,6-trimethyl-, (1 S-(1alpha,3(2E,4E,6S*),5 alpha, 5(1 E,3E,5E),6 alpha)) or Manumycin A or also called UCF1-C, described in EP456474 (Kyowa Hakko Kogyo KK (JP)).
N-Acetyl-N-naphthylmethyl-2(S)-[(1-(4-cyanobenzyl)-1H-imidazol-5-yl)acetyl]amino-3(S)-methylpentamine, described in WO9639137 (Merck & Co Inc. (US)).
4,9-Ethano-3aH-benz[f]isoindole-3a-carboxylicacid, 1,2,3,4,9,9a-hexahydro-2-[2-(2-methoxyphenyl)-1-oxo-2-propenyl]-9-(4-methylphenyl)-, (3aR,4S,9S,9aR) or also identified as RPR-130401 and described in WO9829390 (Rhône Poulenc Rorer SA (FR) - Sanofi-Aventis). RPR-130401 has the following formula: 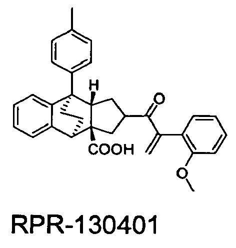
(1alpha,2beta,3beta,4alpha)-1,2-di[N-Propyl-N-(4-phenoxybenzyl)aminocarbonyl]cyclobutane-3,4-dicarboxylate, also identified as A-87049 and described in WO9634851 (Abbott Lab. (US)).
1-Cyclohexene-1-methanol, 4-(1-methylethenyl), also named perillyl alcohol and described in US5110832 (Chastain Doyle E (US) - DOR BioPharma). This compound has the following formula: 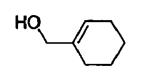
Cys-Val-Phe-Met (or CVPM, Bristol-Myers Squibb) and described in Reiss, Y., Goldstein, J. L., Seabra, M. C., Casey, P. J. and Brown, M. S. (1990) Cell 62, 81-88. CVPM presents the following formula: 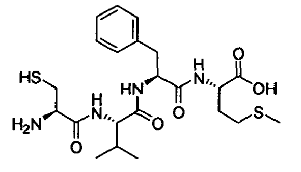
(S)-4-(5-{[1-(3-Chlorobenzyl)-2-oxopyrrolidin-3-ylamino]methyl}imidazol-1-ylmethyl)benzonitrile described in Bell, I. M., J. Med. Chem., 2001, 44, 2933. (PTFase IC50 = 1.9 nM). The above compound has the following formula: 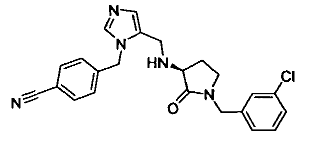
FTI-232 (Cys-4-ABA-Met), FTI-205 and FTI-249 described in Quian Y., et al., J. Biol. Chem., 269, 12410, 1994. (FTI-232: PTFase IC50 = 50 nM, FTI-249: PTFase IC50 = 50 nM). These compounds present the following formulas: 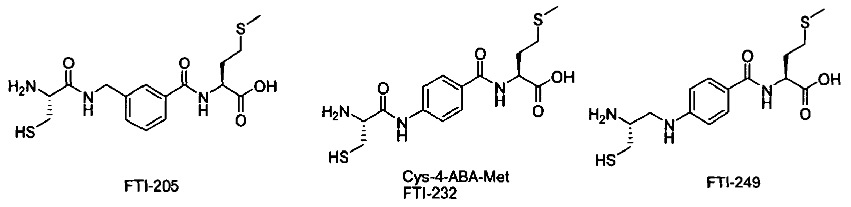
FTI-2287 and FTI-2312 described in Ohkandha, J., J. Med. Chem., 45, 177, 2002. (FTI-2312: PFTase IC50 = 430 nM). These compounds present the following formulas: 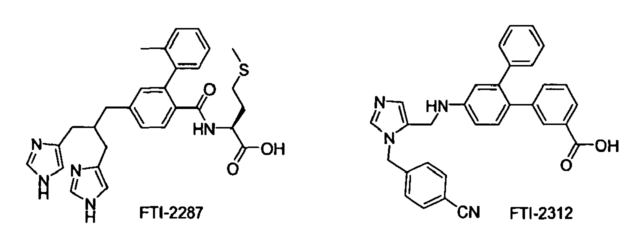
J-104,134 and J-104,135 described in Aoyama, T. et al., J. Med. Chem., 41, 143, 1998 (Banyu Pharmaceuticals) (J-104,134: PTFase IC50 = 5 nM; J-104,135: PTFase IC50 = 3.9 nM). J-104,134 and J-104,135 present the following formulas: 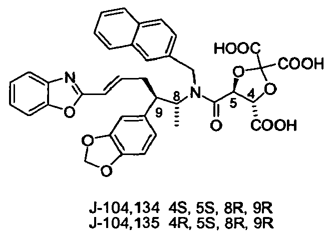
BZA-2B, BZA-4B and BZA-5B described in Stadley, S. J. et al., Biochemistry, 32, 12586, 1993; James, G.L. et al., Science, 260, 1937, 1993 (Genentech) (BZA-2B: PFTase IC50 = 0.85 nM; BZA-4B: PFTase IC50 = 1.3 nM; BZA-5B: PFTase IC50 = 41 nM). These compounds present the following formulas: 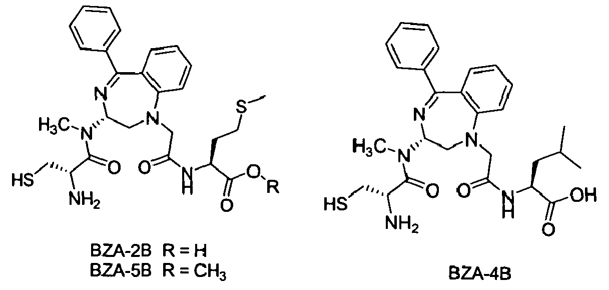
L-739,750 and L-739,749 described in Kohl, N. E. et al., Proc. Natl. Acad. Sci. USA, 91, 9141, 1994 (Merck) (L-739,750: PFTase IC50 = 1.8 nM). These compounds present the following formulas: 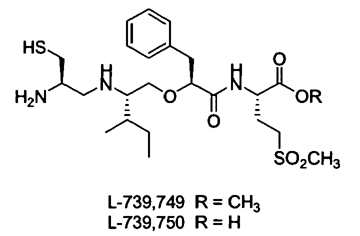
(R*)-N-[[1,2,3,4-Tetrahydro-2-[N-[2-(1H-imidazol-4-yl)ethyl]-L-valyl]-3-isoquinolinyl]carbonyl]-L-methionine ([imidazol-4-yl-ethyl]-Val-Tic-Met) or BMS-193269 described in Hunt, J.T., J. Med. Chem., 39, 353, 1996 (BMS-193269: FTase IC50 = 0.79 nM). This compound has the following formula: 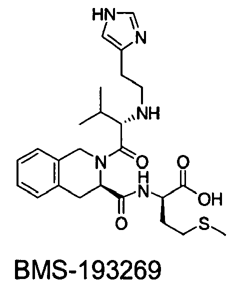
RPR 113829 and its methyl ester prodrug RPR 114334 described in Clerc F. F. et al., Bioorg & Med. Chem. Lett 5, 1779, 1995 (Rhône Poulenc Rorer) (RPR 113829: FTase IC50 = 1.8 nM). These compounds have the following formulas: 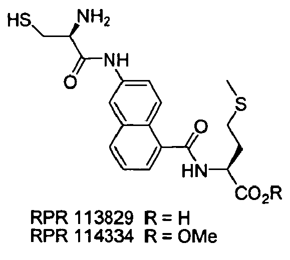
B956 and its methyl ester B1086 described in Nagasu et al., Cancer res., 55, 5310, 1995 (Eisaï) (B956: PFTase IC50 = 11 nM). These compounds have the following formulas: 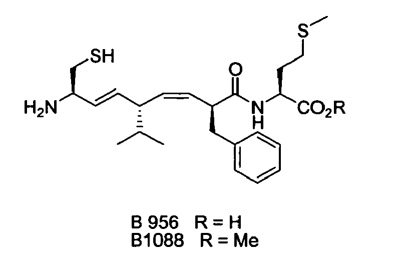
BMS-186511 described in Patel, D. V. et al., J. Med Chem., 38, 2906, 1995 (BMS-186511: PFTase IC50 = 10 nM). This compound has the following formula: 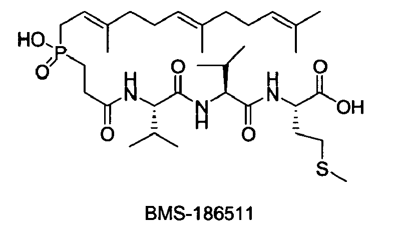
Methyl N-benzoyl-N-(piperidin-4-yl-N-(R)-cysteinyl)-(S) methioninate described in Houssin R. et al., J. Med. Chem., 45, 533, 2002. (PFTase IC50 = 20 nM). This compound has the following formula: 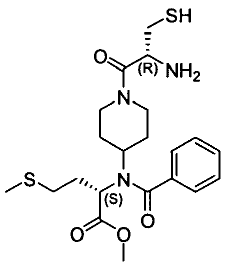
N-[3-Benzoyl-4-[(4-methylphenyl)acetylamino]phenyl]-5-phenylvaleryl amide described in Böhm M. et al., J. Med. Chem. 44, 3117, 2001 (PFTase IC50 = 390 nM). This compound has the following formula: 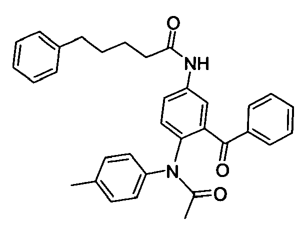
(+)-4-(4-Chloro-3,6,7,12-tetrahydro-1-methylpyrido[2',3':4,5]cyclohepta-[2,1-e]indol-12-yl)-1-(4-pyridinylacetyl)piperidine N1-Oxide (or Sch-207758) described in Taveras A. G. et al., J. Med. Chem. 44, 3117, 2001 (Sch-207758: PFTase IC50 = 7.4 nM). This compound has the following formula: 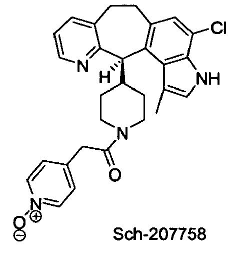
(+)-4-(2-Bromophenyl)-2-(3,4-dihydroxyphenyl)-3-nitro-1-(3-pyridylmethyl)piperidine described in Nara S. et al., J. Med. Chem., 46, 2467, 2003 (PFTase IC50 = 1.9 nM). This compound has the following formula:

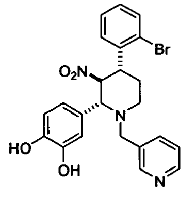

Most preferred GGT inhibitors for use in the present invention are:
L-Leucine, N-[4-[[(2R)-2-amino-3-mercaptopropyl]amino]-2-(1-naphthalenyl)benzoyl]-, methyl ester (or GGTI-298) and the corresponding acid (GGTI-297) cited in McGuire T.F. et al., J. Biol. Chem. 271, 27402, 1996 (GGTI-297: PGGTase-I IC50 = 50 nM). These compounds have the following formulas: 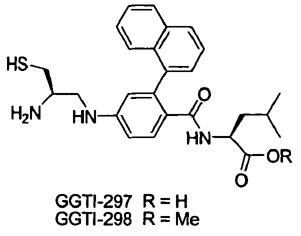
L-Leucine, N-[[5-[[(2R)-2-amino-3-mercaptopropyl]amino][1,1'-biphenyl]-2-yl]carbonyl]-, methyl ester (or GGTI-286) and its corresponding acid (GGTI-287) cited in Lerner E. C. et al., J. Biol. Chem., 270, 45, 26770, 1995 and Lerner E. C. et al., J. Biol. Chem., 270, 45, 26802, 1995 (GGTI-287: PGGTase-I IC50 = 5 nM). These compounds have the following formulas: 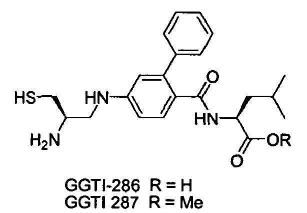
4-((5-((4-(3-chlorophenyl)-3-oxopiperazin-1-yl)methyl)-1H-imidazo)-1-yl)methyl)-2-phenoxybenzonitrile (Merck & Co). This compound has the following formula: 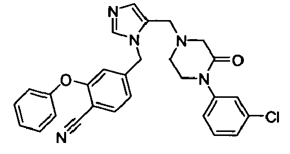
GGTI-2154 described in Vasudevan, A. et al., J. Med. Chem., 42, 1333, 1999 (GGTI-2154: PGGTase IC50 = 21 nM). GGTI-2166 cited in Sun, J. et al., Cancer Res., 59, 4919, 1999. These compounds have the following formulas:

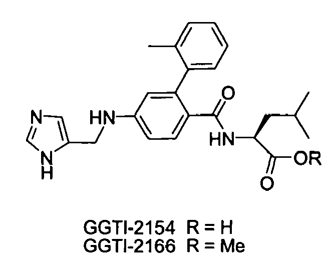

The present invention also includes, as prenylation inhibitors, the optical and geometrical isomers, racemates, tautomers, salts, hydrates and mixtures of the above cited compounds.

Also, it should be understood that the present invention is not limited to the compounds identified above, but shall also include any compound and derivative thereof cited in the references mentioned above, as well as all farnesyl or geranyl inhibitors (FTIs or GGTI_{S}) known to the man skilled in the art, which are appropriate for use in human subjects.

Furthermore, the prenyl inhibitors also include prodrugs of compounds cited above which, after administration to a subject, are converted to said compounds. They also include metabolites of compounds cited above which display similar therapeutic activity to said compounds.

### Formulation and administration

The FTIs or GGTIs according to the invention may be formulated in any appropriate medium or formulation or composition suitable for use in human subjects. Typically, such formulations or compositions include pharmaceutically acceptable carrier(s) or excipient(s), such as isotonic solutions, buffers, saline solution, etc. The formulations may include stabilizers, slow-release systems, surfactants, sweeteners, etc. Such formulations may be designed for various administration routes, including systemic injection (e.g., intravenous, intracerebral, intramucular, transdermic, etc.) or oral administration.

The compositions may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.

Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.

Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

The compounds may be formulated in various forms, including solid and liquid forms, such as injectable solutions, capsules, tablets, gel, solution, syrup, suspension, powder, etc.

In a particular embodiment, the FTIs or GGTIs according to the invention are incorporated into a specific pharmaceutical formulation or technology that enables their delivery to the human brain using catalysed-transport systems.

Specific pharmaceutical formulations include, for instance, suitable liposomal carriers to encapsulate neuroactive compounds that are stable enough to carry them to the brain across the BBB with the appropriate surface characteristics for an effective targeting and for an active membrane transport.

Specific technologies include, for instance, suitable nanoparticle-based brain drug delivery systems to deliver drugs to the brain. These systems mask the BBB-limiting characteristics of the drug, enable targeted brain delivery via BBB transporters and provide a sustained release in brain tissue which could reduce dosage frequency, peripheral toxicity, and adverse effects.

Other suitable pharmaceutical formulations are disclosed in the prior art literature, such as in US5,874,442 ; WO01/46137 ; WO97/30992 ; WO98/00409 or WO97/17070, for instance, which are incorporated therein by reference.

Appropriate dosages and regimens may be determined by the skilled artisan, based on the present description and the available prior art literature. In particular, repeated administrations may be performed, with dosages ranging from 0.001 to 100 mg.

The invention allows effective treatment of Parkinson's Disease, e.g., a reduction in symptoms, disease progression, muscular rigidity or tremor. The treatment may be carried out using any such FTI or GGTI, either alone or in combination(s), optionally combined to other therapeutically active agents.

### Products and Diagnosis

As discussed above, the present invention also discloses a novel metabolic pathway involved in neuroprotection. Furthermore, the invention show that genetic alterations occur within members of this pathway, which represent valuable therapeutic targets, e.g., for drug screening or disease diagnosis, as well as for use as active agents or immunogens.

In this context, the invention particularly describes the appearance of alternative forms of the mRNA encoding Rac1 or RhoB in neuronal cells subjected to oxidative stress, and particularly of forms altered at the level of the last exon and/or 3'UTR. Other forms can be envisioned and investigated within the scope of the present application.

Accordingly, the present invention relates to methods of detecting the presence or predisposition to oxidative stress comprising detecting, in a sample from a subject, the presence of an altered Rac1 or RhoB locus, the presence of such altered locus being indicative of the presence or predisposition to oxidative stress.

A further object of this invention is a method of selecting drugs, comprising a step of determining whether a candidate drug can alter Rac1 or RhoB locus, e.g., the (relative) amount of splicing forms of said gene(s).

Within the context of the invention, the term Rac1 or RhoB locus denotes any sequence or any Rac1 or RhoB product in a cell or an organism. This term particularly means the nucleic acid sequences, either coding or non-coding, as well as the protein sequences, whether mature or not. Therefore, the term Rac1 or RhoB locus includes all or part of the genomic DNA, including its coding and/or non-coding regions (introns, regulatory sequences, etc.), the RNA (messenger, pre-messenger, etc.) and the Rac1 or RhoB proteins (precursor, mature, soluble, secreted, etc. forms), present in an organism, tissue or cell.

The term "Rac1 gene" or "RhoB gene" denotes any nucleic acid encoding a Rac1 or RhoB polypeptide. It can be genomic (gDNA), complementary (cDNA), synthetic or semi-synthetic DNA, mRNA, synthetic RNA, etc. It can be a recombinant or synthetic nucleic acid, produced by techniques known to those skilled in the art, such as artificial synthesis, amplification, enzymatic cleavage, ligation, recombination, etc., using biological sources, available sequences or commercial material. A Rac1 or RhoB gene exists typically in a two-stranded form, even though different forms can exist according to the invention. The sequence of the Rac1 gene is available in certain data banks, such as, notably, RefSeq, n° NM_009007. The sequence of the RhoB gene is also available in certain data banks, such as, notably, RefSeq, n° NM_022542. Other Rac1 or RhoB gene sequences, according to the invention, can be isolated from samples, or collections, or may be synthesized. Rac1 sequences can relate to sequences that hybridize in highly stringent conditions with a nucleic acid encoding the sequence SEQ ID NO: 8 presented below. Similarly, RhoB sequences can relate to sequences that hybridize in highly stringent conditions with a nucleic acid encoding the sequence SEQ ID NO: 9 presented below.

The term Rac1 polypeptide particularly denotes any polypeptide encoded by a Rac1 gene as defined herein above. A specific example is supplied below (SEQ ID NO: 8), corresponding to the sequence referenced in Genbank under the number NP_033033.1.

The term Rac1 polypeptide also includes, in the broad sense, any biologically active natural variant of the sequence identified above that could result from polymorphisms, splicing, mutations, insertions, etc.

The term RhoB polypeptide particularly denotes any polypeptide encoded by a RhoB gene as defined herein above. A specific example is supplied below (SEQ ID NO: 9), corresponding to the sequence referenced in Genbank under the number NP_071987.1.

Alteration of the rac1 or rhoB locus can be of a diverse nature, such as, in particular, one or several mutations, insertions, deletions and/or spicing events or the like, in the gene or RNA encoding Rac1 or those encoding RhoB . Advantageously it is a splicing event, for example the appearance of a splice form of Rac1 or RhoB or modification of the ratio between different splice forms or between a non-spliced form and spliced forms.
In more preferred embodiments, the above methods comprise detecting the presence of an altered splicing of Rac1 or RhoB, e.g., the appearance of particular splicing isoforms or the presence of an altered ratio between splicing isoforms. More specifically, the method comprises detecting the presence of a nucleic acid molecule comprising SEQ IS NO: 1, 2, 3, 4, 5, 6, 7, 10, 11, 12, 13, 14, 15 or 16, or a corresponding polypeptide. Such nucleic acid molecules and polypeptides also represent particular object of the present invention, as well as any distinctive fragment or analogs thereof ; antibodies specifically binding to such polypeptides and specific nucleic acid probes or primers.

Further aspects and advantages of this invention will be disclosed in the following examples, which should be regarded as illustrative and not limiting the scope of this application. All cited publications or applications are incorporated therein by reference in their entirety.

### EXAMPLES

### Example 1: Identification of DATAS signatures from MPTP treated dopaminergic neurons

In order to identify the repertoire of splicing events and any other qualitative modifications of mRNA, the patented DATAS technique was applied to mRNA from control dopaminergic mesencephalic cultures and those treated with the neurotoxin MPTP/MPP+ (1-methyl-4-phenylpyridinium (MPP+)). The resulting action of MPTP is the increase in production of reactive oxygen species within the cells and the subsequent selective apoptosis of tyrosine hydroxylase-positive dopaminergic neurons.

Rat mesencephalon neurons are cultured according to the previously described method of Schinelli et al. (Schinelli et al. 1998) with some minor modifications. Half of cultures are incubated on day 6 with MPP+ at 4 µM for 48 h, the other untreated cultures are used as controls.

### Identification of an alteration of Rac1

Among the clones identified were 5 fragments of mRNA corresponding to a mouse homolog of *Rattus norvegicus* RAS-related C3 botulinum substrate 1 (Rac1). The DATAS fragments are : EXR-NPDA1209-01, length: 515 (SEQ ID NO: 1), EXR-NPDA1225-01 (SEQ ID NO: 2), length 507, EXR-NPDA1226-01, length 507 (SEQ ID NO: 3), EXR-NPDA1237-01, length 515 (SEQ ID NO: 4), EXR-NPDA1256-01, length 523 (SEQ ID NO: 5). The conserved region in these DATAS fragments corresponds to nucleotides 611 to 1219 of the RefSeq bank sequence, referenced under the number NM_009007. It corresponds to the last exon and the 3'UTR of Rac1 because Rac1 CDS stops at position 776.

### Identification of an alteration of RhoB

Among the clones identified were also 2 fragments of mRNA corresponding to *Rattus norvegicus* rhoB gene (Arhb) The DATAS fragments are EXR-NPDA0544-01 length: 515 (SEQ ID NO: 6) and RHOB DATAS fragment EXR-NPDA0565-01 length: 518 (SEQ ID NO: 7). The conserved region in these two DATAS fragments corresponds to nucleotides 678 to 1200 of the RefSeq bank sequence, referenced under the number NM_022542. It corresponds to the last exon and the 3'UTR of RhoB because the coding sequence stops at position 873.

The identification of these fragments indicates a novel deregulation of Rac1 and RhoB at the level of alternative splicing occurring within the last exon and the 3'UTR in neuronal cells intoxicated with MPTP compared to control cells.

Searches in various public ETSs databases such as Genbank, DDBJ (DNA Data Bank of Japan), and EMBL (European Molecular Biology Laboratory) using publicly available bioinformatic tool such as BLAT (Kent, 2002) failed to identify any additional alternative splicing event in the considered regions These novel modifications of the mRNA likely affect either the length of the last exon of Rac1 and RhoB, or the 3'UTR region that contains sequences involved in mRNA stability or translational control.

Thus, the above modifications of Rac1 and RhoB locus likely affect Rac1 or RhoB C-terminal structure, protein activity and levels in situations where a selective death of dopaminergic, tyrosine hydroxylase-positive, neurons is induced by MPTP.

### Identification of an alteration of GEF

Among the clones identified were also 6 fragments of mRNA corresponding to a mouse homolog of *Rattus norvegicus* Rap guanine nucleotide exchange factor (GEF) 4 (Rapgef4). The DATAS fragments are : SEQ ID NO: 10 : DATAS fragment EXR-NPDA1726-01 length 515; SEQ ID NO: 11 : DATAS fragment EXR-NPDA1726-01, length 515; SEQ ID NO: 12 : DATAS fragment EXR-NPDA1726-01 length 515; SEQ ID NO: 13 : DATAS fragment EXR-NPDA1726-01 length 515; SEQ ID NO: 14 : DATAS fragment EXR-NPDA1775-01 length 507; SEQ ID NO: 15 : DATAS fragment EXR-NPDA1780-01 length 506.

The conserved region in these DATAS fragments corresponds to nucleotides 1328 to 2173 of the RefSeq bank sequence, referenced under the number NM_019688. It corresponds to the CDS and likely represents alterations in the coding sequence due to exon skipping or intron retention.

### Identification of an alteration of PAK1

Among the clones identified were also one fragment of mRNA corresponding to *Rattus norvegicus* p21 (CDKN1A) -activated kinase 1 (Pak1). The DATAS fragments is : SEQ ID NO: 16 : DATAS fragment EXR-NPDA1756-01 (length 522). The DATAS fragments corresponds to nucleotides 1474 to 1968 of the RefSeq bank sequence, referenced under the number NM_017198. It corresponds to the CDS and likely represents alterations in the coding sequence due to exon skipping or intron retention.
For both GEF and PAK1, searches in public ETSs databases Genbank, DDBJ (DNA Data Bank of Japan), and EMBL (European Molecular Biology Laboratory) using publicly available bioinformatic tool such as BLAT failed to identify any additional alternative splicing event in the considered region.

### Example 2: Protection of dopaminergic neuronal cells against 6-OH dopamine induced toxicity by farnesyl transferase inhibitor L-744,832

Parkinson's disease (PD) is a progressive neurodegenerative disorder characterized by a loss of nigrostriatal neurons, which results in a severe depletion of dopamine (DA) levels in the basal ganglia.

The catecholamine-specific neurotoxin 6-hydroxydopamine (6-OHDA) is a hydroxylated analogue of DA that leads to apoptosis of catecholaminergic cells. This neurotoxin is classically used to create animal models of Parkinson's disease by either a unilateral injection of 6-OHDA into the medial forebrain bundle or the substantia nigra pars compacta, which results in a rapid degeneration of the nigrostriatal pathway, or as an injection of 6-OHDA into the striatum, which produces a progressive degeneration (>1 week) of the nigrostriatal pathway. This latter paradigm is believed to more closely resemble the natural pathology of PD (Sauer and Oertel 1994).

The toxic effect of 6-OHDA is thought to be mediated by uptake into catecholaminergic nerve endings through the high affinity catecholamine transporter systems. The neurotoxin probably induces cell death by three main mechanisms: (1) reactive- oxygen species (ROS) generation by auto-oxidation, (2) hydrogen peroxide generation after deamination by monoamine oxidase and/or (3) direct inhibition of mitochondrial complexes I and IV (Cohen and Heikkila 1974 and Glinka and Youdim 1995). Some evidence exists that 6-OHDA can be considered as a physiological endogenous neurotoxin, as previously reported in both rat (Senoh and Witkop 1958) and human brain (Curtius et al. 1974; Jellinger et al. 1995; Linert et al. 1996). A non-enzymatic reaction between dopamine, hydrogen peroxide, free iron and manganese elements, which are all found in higher amounts in PD brains, may possibly lead to 6-OHDA formation (Slivka and Cohen 1985; Kienzl et al. 1999; Kienzl et al. 1995).

6-Hydroxydopamine is able to induce apoptosis in various catecholaminergic cells types, such as pheochromocytoma cells (PC12) (Nie et al. 2002), human neuroblastoma cells SK-N-SH (Shimizu et al. 2002), chromafin cells (Galindo et al. 2003) or primary cultures of mesencephalic neurons (Michel and Hefti 1990; Pong et al. 2000; Ding et al. 2004).

SKNSH sub-clone SH-SY5Y is a widely accepted model to study 6-OHDA neurotoxicity and neuroprotection (Zuo et al. 1995; Storch et al. 2000; von Coelln et al. 2001). SH-SY5Y cells can be differentiated into post-mitotic dopaminergic neuronal cells by retinoic acid plus BDNF or TGFbeta treatments and are easier to culture and propagate compared to primary cells. In these cells, 6-OHDA induces apoptosis and toxicity is associated with ROS production, oxidative stress but also protein degradation and ubiquitinproteasome system activation (for reviews, see Youdim et al. 2001; Maruyama et al. 2002; Elkon et al. 2001).

To test whether inhibition of Ras farnesylation could protect SH-SY5Y cells against 6-OHDA induced oxidative stress, a Ras farnesyltransferase inhibitor, L-744,832, was used in 6-OHDA treated SH-SY5Y cells.

L-744,832 ((2S)-2-[[(2S)-2-[[(2S,3S)-2-[[(2R)-2-Amino-3-mercaptopropyl]amino]-3-methylpentyl]oxy]-1-oxo-3-phenylpropyl]amino]-4-(methylsulfonyl)-Butanoic acid 1-methylethyl ester) inhibits in vivo p70s6k phosphorylation in mammary tumors, and induces tumor regression in transgenic mice by mediating alterations in both cell cycle control and apoptosis (Law et al. 2000). The reported active concentration for L-744,832 is generally within 25-60µM concentration range in cell-based assays (Law et al. 2000; Birkenkamp et al. 2004)

SH-SY5Y cells were plated in 24 well plates (ATGC, France) at the initial density of 3*10⁵ cells/well. After 24 hours, cells were pretreated with L-744,832 for 6 hours at concentrations ranging from 20 nM to 40 µM. Then, 6-OHDA (Sigma) diluted in PBS was added to the wells at the concentration of 50µM. After 17 h incubation, an MTT assay was conducted to reveal cell viability, which was measured as normalized optical densities values (treated cells/untreated control cells), where a value of 1 represents 100 % neuronal survival and a value of 0.6 represents 60% survival.

L-744,832 demonstrated a 53% survival ratio, compared to the control, where a 38% survival ratio was found. Thus, L-744,832 exhibited a protective effect of 24% in the case of an overnight treatment with 6-OHDA (Figure 1). Therefore, L-744,832 is a potential neuroprotective agent, in vitro, against cell death induced by ROS. Interestingly, L-744,832 is non toxic for SH-SY5Y cells and the protective activity is only detected at concentrations which were demonstrated to affect protein farnesylation in cells (Law et al. 2000; Birkenkamp et al. 2004).

### Bibliography

Anderson. *Curr Treat Options Neurol.* 6(3):201-207, 2004
Aoyama et al. *J. Med. Chem.* 41:143, 1998
Aznar and Lacal. *Cancer Lett.* 165(1):1-10, 2001
Bell *J. Med. Chem.* 44:2933, 2001
Birkenkamp et al. *Leukemia.* 18:103-112, 2004
Böhm et al. *J. Med. Chem.* 44:3117, 2001
Clerc et al. *Bioorg & Med. Chem. Lett.* 5:1779, 1995
Cohen and Heikkila. *J. Biol. Chem.* 249(8):2447-2452, 1974
Crul et al. *Anticancer Drugs.* 12(3):163-84, 2001
Curtius et al. *J. Chromatogr.* 99(0):529-40, 1974
Ding et al. *J. Neurochem.* 89(3):776-787, 2004
Elkon et al. *Cell. Mol. Neurobiol.* 21(6):771-781, 2001
Galindo et al. *J. Neurochem.* 84(5):1066-1073, 2003
Gash et al. *Nature.* 380(6571 ):252-5, 1996
Gill et al. *Nat Med.* 9(5):589-95, 2003
Glinka and Youdim. *Eur. J. Pharmacol.* 1995
Houssin et al. *J. Med. Chem.* 45:533, 2002
Hunt et al. *J. Med. Chem.* 43:3587-3595, 2000
Hunt *J. Med. Chem.* 39:353, 1996
James et al. *Science.* 260:1937, 1993
Jellinger et al. *J. Neural. Transm. Suppl.* 6:297-314, 1995
Kent, *Genome Res.* 12: 656-664, 2002
Kienzl et al. *Life Sci.* 65(18-19):1973-1976, 1999
Kienzl et al. *J. Neurol. Sci.* 134 Suppl:69-78, 1995
Kohl et al. *Proc. Natl. Acad. Sci. USA.* 91:9141, 1994
Law et al. *J. Biol. Chem.* 275:10796, 2000
Le Wang et al. *J. Med. Chem.* 47:612, 2004
Lerner et al. *J. Biol. Chem.* 270(45):26770, 1995
Lerner et al. *J. Biol. Chem.* 270(45):26802, 1995a
Lin et al. *Science.* 260(5111):1130-2, 1993
Linert et al. *Biochim. Biophys. Acta.* 1316(3):160-168, 1996
Lobell *Mol. Cancer Ther.* 1:747, 2002
Louis et al. *Arch Neurol.* 61(8):1273-6, 2004
Maruyama et al. *Neurotoxicol. Teratol.* 24(5):675-682, 2002
Maurer-Stroh et al. *Biol Chem.* 384(7):977-89, 2003
McGuire et al. *J. Biol. Chem.* 271:27402,1996
Michel and Hefti. *J. Neurosci. Res.* 26(4):428-35, 1990
Nagasu et al. *Cancer Res.* 55:5310, 1995
Nara et al. *J. Med. Chem.* 46:2467, 2003
Nie et al. *Arch. Biochem. Biophys.* 397(1):84-90, 2002
Ohkanda et al. *Prog Cell Cycle Res.* 5:211-7, 2003
Ohkandha *J. Med. Chem.* 45:177, 2002
Olanow and Tatton. *Annual Review of Neuroscience.* Vol. 22: 123-144, 1999
Pahnke et al. *Exp Cell Res.* 297(2):484-94, 2004
Patel et al. *J. Med Chem.* 38:2906, 1995
Pong et al. *Brain Res.* 881(2):182-189, 2000
Quian et al. *J. Biol. Chem.* 269:12410, 1994
Reiss et al. *Cell* 62:81-88, 1990
Ridley. *Traffic.* 2(5):303-10, 2001
Sauer and Oertel. *Neuroscience.* 59(2):401-15, 1994
Schinelli S et al. *J. Neurochem.* 50(6):1900-1907, 1998
Senoh and Witkop. *J. Biol. Chem.* 233(3):697-701, 1958
Shimizu et al. *Neuropharmacology.* 43(3):434-43, 2002
Slivka and Cohen. *J. Biol. Chem.* 260(29):15466-15472, 1985
Stadley et al. *Biochemistry.* 32:12586, 1993
Storch et al. *J. Neural. Transm.* 107(3):281-93, 2000
Sun et al. *Cancer Res.* 59:4919, 1999
Tabner et al. *Curr Top Med Chem.* 1(6):507-17, 2001
Taveras et al. *J. Med. Chem.* 44:3117, 2001
Trapp et al. *Mol Cell Neurosci.* 17(5):883-94, 2001
Vasudevan et al. *J. Med. Chem.* 42:1333, 1999
von Coelln et al. *J. Neurochem.* 77(1):263-73, 2001
Youdim et al. *Cell. Mol. Neurobiol.* 21(6):555-573, 2001
Zuo et al. *Prog. Brain Res.* 106:199-205, 1995

## Claims

1. The use of a protein prenylation inhibitor for the manufacture of a medicament for treating Parkinson's Disease.

2. The use of claim 1, wherein the protein prenylation inhibitor is a geranylgeranyltransferase (GGT) or a farnesyltransferase (FT) inhibitor.

3. The use of claim 1 or 2, for the manufacture of a medicament for protecting neurons from oxidative stress in a subject having Parkinson's Disease.

4. The use of any one of claims 1 to 3, for the manufacture of a medicament for protecting dopaminergic neurons in a subject having Parkinson's Disease.

5. The use of any one of the preceding claims, wherein the inhibitor is a compound having an IC50 for GGT or FT that is below about 1 mM, preferably below 50 nM.

6. The use of any one of the preceding claims, wherein the inhibitor is selective for FT or GGT.

7. The use of any one of the preceding claims, wherein the inhibitor crosses the blood-brain barrier.

8. The use of any one of the preceding claims, wherein the inhibitor is a compound having a molecular weight below about 800 daltons.

9. The use of any one of the preceding claims, wherein the inhibitor is a FT inhibitor compound selected from:
. 6-[Amino(4-chlorophenyl)-1-methyl-1H-imidazol-5-ylmethyl]-4-(3-chlorophenyl)-1-methyl-2(1H)-quinolinone;
. 4-(3-chlorophenyl)-6-[(4-chlorophenyl)hydroxy(1-methyl-1H-imidazol-5-yl)methyl]-1-methyl-2(1H)-quinolinone;
. 6-[(4-chlorophenyl)hydroxy(1-methyl-1H-imidazol-5-yl)methyl]-4-(3-ethoxyphenyl)-1-methyl-2(1H)-quinolinone;
. 6-[(4-chlorophenyl)(1-methyl-1H-imidazol-5-yl)methyl]-4-(3-ethoxyphenyl)-1-methyl-2(1 H)-quinolinone monohydrochloride monohydrate;
. 6-[amino(4-chlorophenyl)(1-methyl-1H-imidazol-5-yl)methyl]-4-(3-ethoxyphenyl)-1-methyl-2(1H)-quinolinone;
. 6-[amino(4-chlorophenyl)(1-methyl-1H-imidazol-5-yl)methyl]-1-methyl-4-(3-propylphenyl)-2(1H)-quinolinone;
. (B)-6-[amino(4-chlorophenyl)(1-methyl-1 H-imidazol-5-yl)methyl]-4-(3-chlorophenyl)-1-methyl-2(1H)-quinolinone;
. (+)-4-[2-[4-(8-Chloro-3,10-dibromo-6,11-dihydro-5H-benzo-[5,6]cyclohepta[1,2-b]-pyridin-11 (R)-yl)-1-piperidinyl]-2-oxo-ethyl]-1-piperidinecarboxamide;
. (R)-7-Cyano-2,3,4,5-tetrahydro-1-(1H-imidazol-4-ylmethyl)-3-(phenylmethyl)-4-(2-thienylsulfonyl)-1H-1,4-benzodiazepine;
. Isopropyl (2S)-2-({2-(4-fluorophenetyl)-5-[({(2S,4S)-4-[(3-pyridinylcarbonyl)sulfanyl]tetrahydro-1H-pyrrol-2-yl}methyl)amino]benzoyl}amino)-4-(methylsufanyl)butanoate;
. 2,3,4,5-Tetrahydro-1-(1H-imidazol-4-ylmethyl)-4-(1-naphthalenylcarbonyl)-1H-1,4-benzodiazepine, hydrochloride;
. 1-(3-Chlorophenyl)-4-[1-(4-cyanobenzyl)-5-imidazolylmethyl]-2-piperazinone;
. 1(R),10(S)-Epoxy-5(S),5(S),7(S)-guaia-3(4),11(13)-dien-6,12-olide;
. L-Methionine, N-[[(4R)-3-[(2S,3S)-2-[[(2R)-2-amino-3-mercaptopropyl]amino]-3-methylpentyl]-5,5-dimethyl-4-thiazolidinyl]carbonyl]-, methyl ester,
. L-Methionine, N-[[5-[[(1H-imidazol-4-ylmethyl)amino]methyl]-2'-methyl[1,1'-biphenyl]-2-yl]carbonyl] and its methyl ester;
. 4-[(4-Cyano-2-arylbenzyloxy)-(3-methyl-3H-imidazol-4-yl)methyl]benzonitriles; 5-cyano-2-[(4-cyanophenyl)-(3-methyl-3H-imidazol-4-yl)methoxymethyl]-N-phenylbenzamides;
. (2S)-2-[[(2S)-2-[[(2S,3S)-2-[[(2R)-2-Amino-3-mercaptopropyl]amino]-3-methylpentyl]oxy]-1-oxo-3-phenylpropyl]amino]-4-(methylsulfonyl)-butanoic acid 1-methylethyl ester;
. 1-[1-[1-(1,3-Benzodioxol-5-ylmethyl)-1H-imidazol-5-ylmethyl]-4-(1-naphthyl)-1 H-pyrrol-3-yl]-1-(4-methyl-1-piperazinyl)methanone;
. 2-(3-Pyridyl)-N-(2,2-diphenyl-ethyl)-N-((cis)-3-sulfanylpyrrolidin-2-ylmethyl)acetamide;
. (7,8-Dichloro-5H-dibenzo[b,e][1,4]diazepin-11-yl)-pyridin-3-yl methylamine;
. (2 alpha)-2-Hydroxy-24,25-dihydroxylanost-8-en-3-one;
. L-erythro-L-Glycero-D-altro-7-trideculo-7,4-furanosonic acid, 2,7-anhydro-3,4-di-C-carboxy-8,9,10,12,13-pentadeoxy-10-methy)ene-12-(phenylmethyl)-,11-acetate 5-(4,6-dimethyl-2-octenoate), [5(2E,4S,6S),7S] or zaragozic acid A;
. 2,4-Decadienamide, N-(5-hydroxy-5-(7-((2-hydroxy-5-oxo-1-cyclopenten-I-yl)amino-oxo-1,3,5-heptatrienyl)-2-oxo-7-oxabicyclo(4.1.0)hept-3-en-3-yl)-2,4,6-trimethyl-, (1 S-(1alpha,3(2E,4E,6S*),5 alpha, 5(1 E,3E,5E),6 alpha));
. N-Acetyl-N-naphthylmethyl-2(S)-[(1-(4-cyanobenzyl)-1H-imidazol-5-yl)acetyl]amino-3(S)-methylpentamine;
. 4,9-Ethano-3aH-benz[f]isoindole-3a-carboxylicacid, 1,2,3,4,9,9a-hexahydro-2-[2-(2-methoxyphenyl)-1-oxo-2-propenyl]-9-(4-methylphenyl)-, (3aR,4S,9S,9aR);
. (1 alpha,2beta,3beta,4alpha)-1 ,2-di[N-Propyl-N-(4-phenoxybenzyl)aminocarbonyl]cyclobutane-3,4-dicarboxylate;
. 1-Cyclohexene-1-methanol, 4-(1-methylethenyl);
. Cys-Val-Phe-Met;
. (S)-4-(5-{[1-(3-Chlorobenzyl)-2-oxopyrrolidin-3-ylamino]methyl}imidazol-1-ylmethyl)benzonitrile;
. (R*)-N-[[1,2,3,4-Tetrahydro-2-[N-[2-(1H-imidazol-4-yl)ethyl]-L-valyl]-3-isoquinolinyl]carbonyl]-L-methionine ([imidazol-4-yl-ethyl]-Val-Tic-Met);
. Methyl N-benzoyl-N-(piperidin-4-yl-N-(R)-cysteinyl)-(S) methioninate;
. N-[3-Benzoyl-4-[(4-methylphenyl)acetylamino]phenyl]-5-phenylvaleryl amide;
. (+)-4-(4-Chloro-3,6,7,12-tetrahydro-1-methylpyrido[2',3':4,5]cyclohepta-[2,1-e]indol-12-yl)-1-(4-pyridinylacetyl)piperidine N1-Oxide; (+)-4-(2-Bromophenyl)-2-(3,4-dihydroxyphenyl)-3-nitro-1-(3-pyridylmethyl)piperidine; and
. compounds of the following formulas: 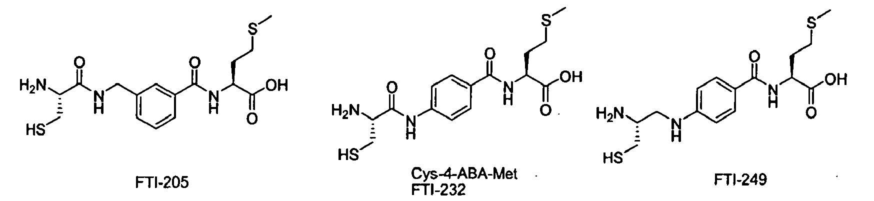 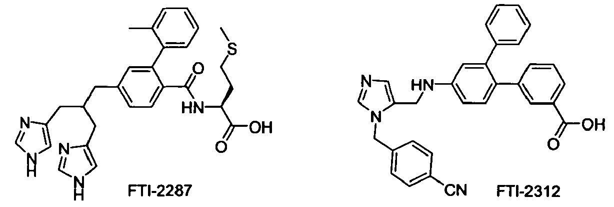 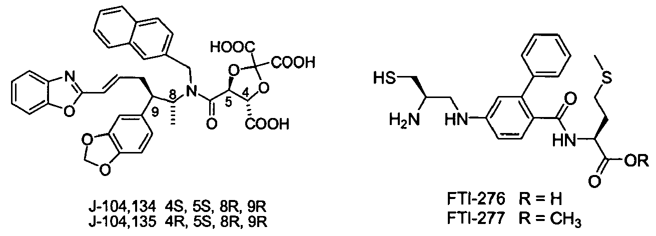 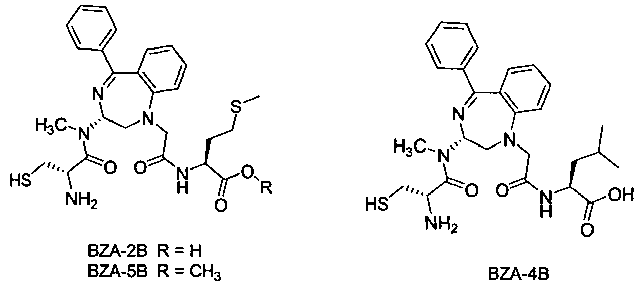 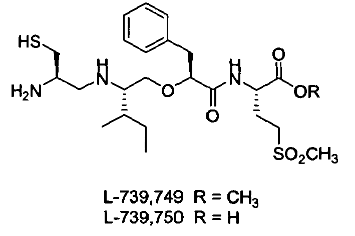 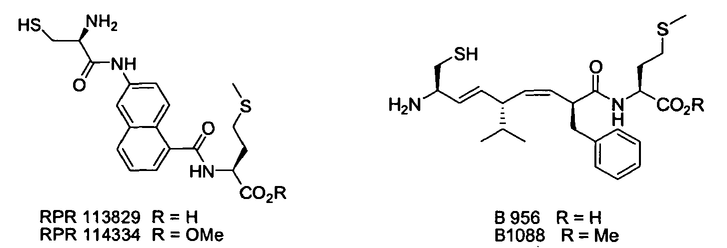 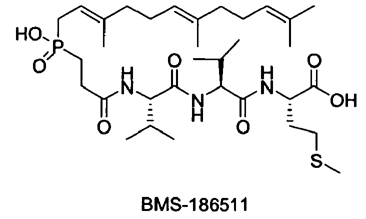
as well as their optical and geometrical isomers, racemates, tautomers, salts, hydrates and mixtures thereof.

10. The use of any one of claims 1 to 8, wherein the inhibitor is a GGT inhibitor compound selected from:
. L-Leucine, N-[4-[[(2R)-2-amino-3-mercaptopropyl]amino]-2-(1-naphthalenyl)benzoyl]-, methyl ester (or GGTI-298) and its corresponding acid (GGTI-297);
. L-Leucine, N-[[5-[[(2R)-2-amino-3-mercaptopropyl]amino][1,1'-biphenyl]-2-yl]carbonyl]-, methyl ester (or GGTI-286) and its corresponding acid (GGTI-287);
. 4-((5-((4-(3-chlorophenyl)-3-oxopiperazin-1-yl)methyl)-1H-imidazol-1 - yl)methyl)-2-phenoxybenzonitrile; and
. compounds of the following formula: 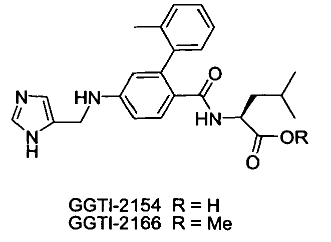 as well as their optical and geometrical isomers, racemates, tautomers, salts, hydrates and mixtures thereof.

11. The use of any one of the preceding claims, wherein the inhibitor is formulated in any pharmaceutically acceptable carrier(s) or excipient(s).

12. The use of claim 11, wherein the inhibitor is incorporated into a specific pharmaceutical formulation or technology allowing delivery to the human brain using catalysed-transport systems.

13. The use of claim 12, wherein said formulation or technology is selected from liposomal carriers and nanoparticles.

14. The use of any one of the preceding claims, wherein the inhibitor is administered to said subject by systemic injection(s) or oral administration(s).

15. The use of any one of the preceding claims, wherein a combination of GGT or FT inhibitors is administered.

16. The use of any one of the preceding claims, wherein the GGT or FT inhibitor(s) is administered in combination with an other active agent.
